Europäisches Patentamt

European Patent Office

Office européen des brevets

(11) Publication number: **0 078 677**
**B1**

## (12) EUROPEAN PATENT SPECIFICATION

(45) Date of publication of patent specification: **03.05.89**

(21) Application number: **82305759.1**

(22) Date of filing: **29.10.82**

(51) Int. Cl.⁴: **A 61 B 5/00,** A 61 B 5/08,
G 01 D 5/20

(54) **Apparatus for monitoring clinically significant surface movements in living organisms.**

(30) Priority: **02.11.81 US 317418**

(43) Date of publication of application:
**11.05.83 Bulletin 83/19**

(45) Publication of the grant of the patent:
**03.05.89 Bulletin 89/18**

(84) Designated Contracting States:
**AT BE CH DE FR GB LI LU NL SE**

(56) References cited:
**DE-A-1 566 044**
**GB-A-1 596 298**
**GB-A-2 062 239**
**GB-A-2 081 454**
**US-A-4 252 129**

(73) Proprietor: **RESPITRACE CORPORATION**
**731 Saw Mill River Road**
**Ardsley New York 10502 (US)**

(72) Inventor: **Sackner, Marvin A.**
**300 West Rivo Alto Drive**
**Miami Beach Florida 33140 (US)**

(74) Representative: **Bowman, Paul Alan et al**
**LLOYD WISE, TREGEAR & CO. Norman House**
**105-109 Strand**
**London WC2R OAE (GB)**

Courier Press, Leamington Spa, England.

## Description

This invention relates to apparatus for monitoring clinically significant surface movements in living organisms, especially humans.

Various types of apparatus are known for measuring surface movements on living organisms, including human subjects. Whilst there are known techniques for measuring intrapleural pressure, one of these techniques measure intrapleural pressure by monitoring movement of the skin surface of the suprasternal fossa.

It is known that a change in cross-sectional area of a conductive loop causes a proportional change in the inductance of the loop. This property has been utilized to monitor variations in a patient's chest and abdomen areas by measuring the inductance of an extensible electrical conductor closely looped around the body and hence monitor respiration volumes of the subject. This method and apparatus therefore are disclosed in British Patent Specification No. 1 596 298. Heretofore it has not been appreciated that by monitoring the inductance and hence the change in area of smaller conductive loops placed upon an area of the body which is subject to surface movement, clinically significant data may be obtained.

United States Patent No. 4 252 129 discloses a device for measuring motion of a living body organ comprising oscillator means including quartz resonator means, two-terminal inductance means for producing an electromagnetic field and capacitor means in series arrangement, said oscillator means being operative to produce an output signal having a frequency in accordance with said resonator means, inductance means and capacitor means respectively, the introduction of said organ into said electromagnetic field causing said field and hence the effective inductance of said two-terminal inductance means to vary in accordance with the proximity of said organ to said inductance means, whereby said effective inductance and hence the frequency of said oscillator means output signal varies in accordance with the motion of said organ relative to said inductance means. The two-terminal inductance means comprises a coil which is embedded in a plastic disc in the detector. This is operated without contacting the skin of the patient.

According to the present invention there is provided apparatus for measuring movement of a portion of the skin surface of a subject comprising:

a conductive loop dimensioned for disposition on the skin surface without enveloping the subject,

means connected to said loop for generating a signal indicative of the inductance of said loop,

in which the loop is adapted such that at least a portion thereof may be secured to the skin surface of the suprasternal fossa of the subject whereby movement of said skin surface causes corresponding movement of said portion of the loop in such a way that the cross-sectional area enclosed by the loop is changed.

Movement of the surface portion being monitored results in proportional movement of the part of the loop lying thereon and hence proportional changes in the cross sectional area of the loop. This, in turn, causes a proportional change in the inductance of the loop. Accordingly, by measuring the changes in the inductance of the loop, the extent of surface movement may be determined. To measure inductance, the conductive loop is preferably incorporated as the inductance element in a variable frequency LC oscillator. Consequently, changes in the loop inductance result in proportional changes in the signal frequency at the output of the oscillator. The variable frequency output signal from the oscillator may then be converted to a corresponding voltage signal suitable for display on a CRT, strip chart recorder, or other suitable output device.

One particularly significant application of the present invention is measuring intrapleural pressure in human subjects. In this application, one part of the conductive loop is secured to the skin surface of the suprasternal fossa, and the remainder of the loop is preferably secured to the skin surface above the manubrium of the sternum. The skin surface of the suprasternal fossa moves in and out as the subject exhales and inhales, whereas the skin surface of the manubrium is relatively free of movement. The loop is connected to suitable circuitry, such as that mentioned above, for monitoring changes in the inductance of the loop. By comparing changes in the inductance of the loop with intrapleural pressure as measured with an esophageal balloon catheter, it was found that at zero flow, i.e. at end expiration and end inspiration, movements of the skin surface of the suprasternal fossa are proportional to changes in intrapleural pressure. Consequently, monitoring changes in the inductance of the loop, which are proportional to these movements, provides a signal proportional to intrapleural pressure. Zero flow points may be determined from simultaneous tidal volume measurements from, e.g. a spirometer.

Furthermore, this signal may be calibrated during an initial calibration procedure by comparing the output signal from the conductive loop at zero flow with intrapleural pressure as measured by an alternative invasive technique, such as the esophageal balloon catheter or preferably a "null technique" to be described hereinafter. Once calibrated, the output signal yields actual intrapleural pressure on a non-invasive basis, provided the subject is relatively immobile. Numerous other applications of the apparatus in accordance with the present invention, some of which will be discussed hereinafter, will suggest themselves to those skilled in the art once this description is known.

The apparatus in accordance with the present invention will be more fully apparent from the following detailed description and annexed drawings of the presently preferred embodiment thereof.

Brief description of the drawings
In the drawings:
Fig. 1 is a perspective view showing, *in situ,* a portion of the apparatus of the present invention for measuring intrapleural pressure;
Fig. 2 is a diagrammatic representation of preferred circuitry for measuring the inductance of the conductive loop in accordance with the invention;
Fig. 3 is a more detailed diagrammatic representation of the demodulating circuit of Fig. 2;
Fig. 4 is a schematic representation of the oscillator circuit of Fig. 2;
Fig. 5 is a graphic representation illustrating the linear relationship between intrapleural pressure and movement of the skin surface of the suprasternal fossa;
Figs. 6A and 6B are graphic representations illustrating one method for calibrating the apparatus of Fig. 2 for measuring intrapleural pressure;
Fig. 7 is a graphic representation illustrating, for a normal subject and a subject with lung disease, the close correlation between dynamic lung compliance based on intrapleural pressure measurements taken with the apparatus of the present invention and dynamic lung compliance based on intrapleural pressure measurements taken with an esophageal balloon catheter;
Fig. 8 is a graphic representation illustrating, for a plurality of normal subjects, the close correlation between dynamic lung compliance based on intrapleural pressure measurements taken in accordance with the apparatus of the present invention and dynamic lung compliance based on intrapleural pressure measurements taken with an esophageal balloon catheter;
Fig. 9 is a graphic representation illustrating, for a plurality of patients with lung disease, the close correlation between dynamic lung compliance based on intrapleural pressure measurements taken in accordance with the apparatus of the present invention and dynamic lung compliance based on intrapleural pressure measurements taken with an esophageal balloon catheter;
Fig. 10 is a graphic representation of static lung compliance based on intrapleural pressure measurements taken in accordance with the apparatus of the present invention for a subject in both the seated and supine positions; and
Fig. 11 is a graphic representation illustrating the ability of the apparatus of the invention to accurately measure intrapleural pressure upon elastic loading of the respiratory system.

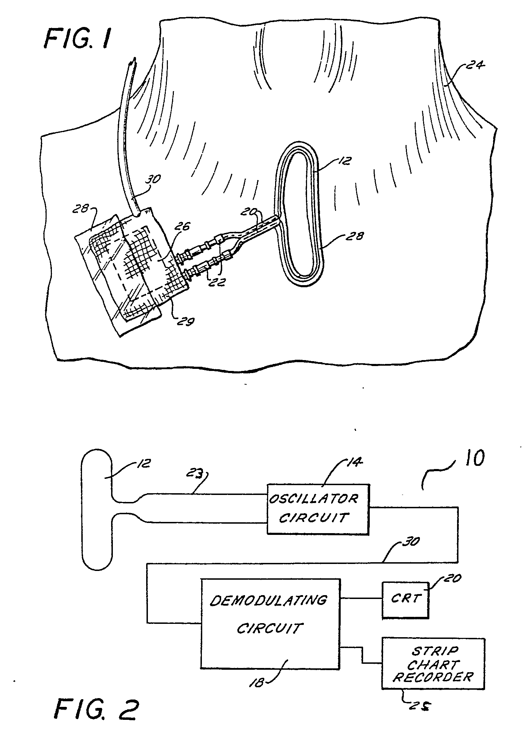

Best mode for carrying out the invention
Referring now to the drawings, and initially to Fig. 2, the preferred apparatus in accordance with the present invention is generally designated by the reference numeral 10. As shown, the apparatus 10 includes a preferably insulated length of conductive wire 12 formed in the shape of a loop. It is known that the inductance of a conductive loop is proportional to the cross sectional area enclosed by the loop. Accordingly, a change in the cross sectional area enclosed by the loop causes a proportional change in the loop inductance.

To apply this technology to monitoring surface movements on living organisms, such as human subjects, the conductive loop is disposed on the subject such that at least a portion of the loop lies on the surface portion being monitored. The loop may be secured in place as by taping, or by employing an adhesive preparation such as a collodion solution, although care should be taken not to inhibit movement of the loop upon movement of the surface portion being monitored.

Movement of the surface portion being monitored causes the loop portion above the moving surface to move. This, in turn, causes a change in the cross sectional area enclosed by the loop and hence in the inductance of the loop. By monitoring these inductance changes in the manner more fully explained below, an indication of the extent of surface movement is provided.

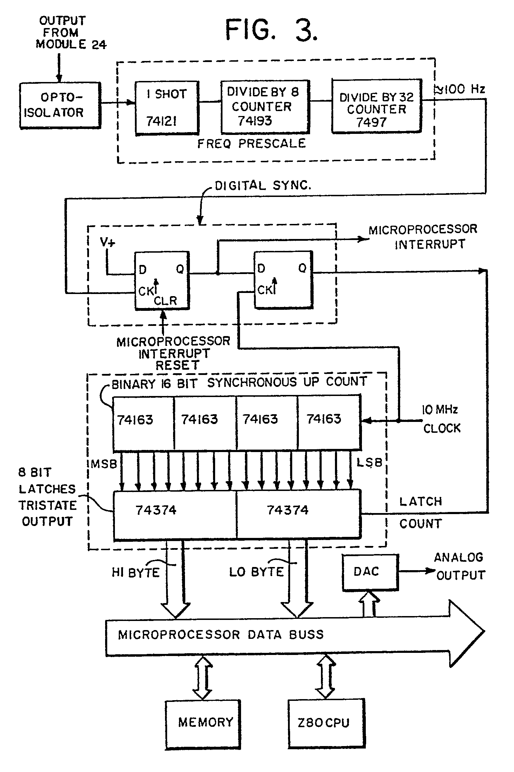

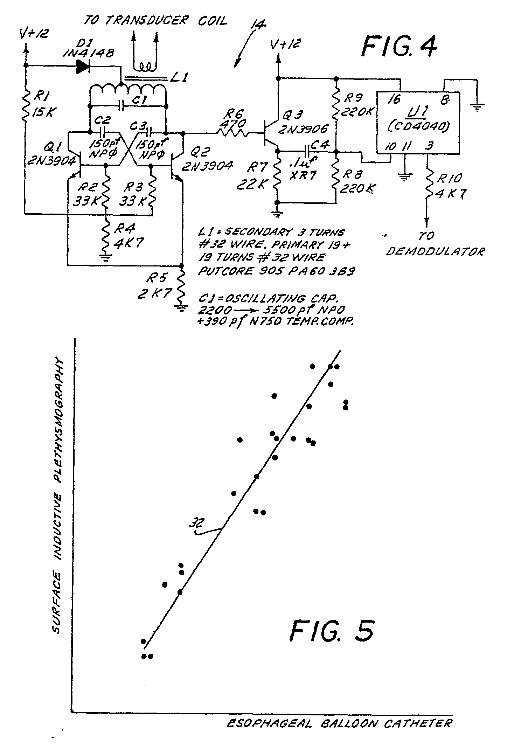

Referring to Figs. 2 and 3, the preferred circuitry for converting the inductance of the loop 12 to a suitable electrical signal is diagrammatically illustrated. As shown, the circuit includes a variable frequency LC oscillator circuit 14 connected to the ends of the conductive loop 12. The resonant frequency of the oscillator circuit 14 is determined by an internal capacitor and the inductance of the conductive loop 12. This frequency may, for example, be centered about 400,000 Hz, and will vary as the cross sectional area enclosed by the loop varies. Because the surface movements being measured are often quite small, it is essential that the oscillator circuit have sufficient sensitivity and gain to measure these movements. A schematic representation of a suitable oscillator circuit 14 is shown in Fig 4. Construction and operation of the oscillator circuit 14 of Fig. 4 will be apparent to those skilled in the art once this description is known. To minimize interference during subsequent signal transmission, and as shown, the oscillator circuit 14 preferably includes an output resistor R10 for converting the oscillator output signal to a current signal.

The output signal from the oscillator circuit 14 is preferably converted to a suitable voltage signal by a demodulator circuit 18. The output of the demodulator circuit 18 is an analog voltage signal having an amplitude which varies in response to variations in the frequency of the oscillator circuit 14. Preferably, the demodulating circuit 18 is digital, the preferred circuitry being diagrammatically illustrated in Fig. 3. The construction and operation of the circuit of Fig. 3 will be apparent to those skilled in the art once this description is known. The analog output signal is provided by a conventional digital to analog converter, and may be displayed on one or more suitable output devices, shown by way of example in Fig. 2 as a CRT terminal 23 and a strip chart recorder 25.

Referring now to Fig. 1 in the drawings, use of the apparatus of the present invention will now be described in connection with a particularly significant application—measurement of intrapleural pressure. Intrapleural pressure, as well as parameters derivable therefrom, is important in the diagnosis of diseased and abnormal lungs. In human subjects, the obvious technique for measuring intrapleural pressure by passing a needle through the chest wall into the pleural space is too dangerous. Instead, the currently accepted technique is to have the subject swallow an esophageal balloon catheter. Because the esophagus is contiguous with the pleural space, the pressure within the balloon reflects intrapleural pressure. This technique, however, is invasive and hence uncomfortable.

To non-invasively measure intrapleural pressure with the apparatus of the invention, the conductive loop 12 is secured on the subject 24 such that one portion of the wire lies on the skin surface of the suprasternal fossa and the other portion of the wire lies on the skin surface of the manubrium of the sternum. Typically, and as shown in Fig. 1, the loop 12 is more or less elliptically shaped and has a circumference of approximately twelve centimeters. The major axis of the ellipse is approximately six centimeters, whereas the minor axis is approximately one and a half to two centimeters. The surface of the suprasternal fossa moves in response to changes in intrapleural pressure, whereas the skin surface of the manubrium is relatively free of movement. Alternatively, the entire loop 12 could be secured to skin surface of the suprasternal fossa, the basic criterion being to ensure that movement of that surface causes a change in the cross sectional area enclosed by the loop.

To minimize artifacts, and as shown in Fig. 1, the oscillator circuit 14 is preferably incorporated in a module 26 secured to the subject 24 in the vicinity of the loop 12. Preferably, both the loop 12 and oscillator module 26 are secured to the subject 24 by tape 28. When taping the loop 12, care should be taken not to inhibit the loop from moving in response to movement of the skin surface of the suprasternal fossa. To this end, a strip of tape 28 in the unstressed shape of loop 12 is used for securing the loop to the subject 24. As shown, a piece of gauze 29 preferably overlies the module 26, and the tape 28 is applied to the gauze for securing the module 26 in place.

A pair of insulated wire leads 20 join the oscillator circuit module 26 to the loop 12, the leads 20 preferably being joined together in the vicinity of the loop. Connectors 22 in the wire leads 20 may be employed to accommodate separation of the loop 12 from the oscillator circuit module 26. It will be apparent that the inductance element of the oscillator 14 is determined not only by the loop 12 but also by the leads 20, and that movement of the leads 20 would therefore be disadvantageous as it would affect the oscillation frequency of the oscillator 14. Accordingly, the leads 20 are preferably substantially rigid, or secured against movement in some other fashion. The leads 20 in Fig. 1 are rendered rigid by the combination of the substantially rigid wire sheaths and connectors 22. A cable 30 extending from the module 26 connects the oscillator circuit 14 to the demodulating circuit 18 and connected output devices 23 and 25.

As the subject exhales and inhales, the changes in intrapleural pressure cause in and out movement of the skin surface of the suprasternal fossa. In accordance with the invention, movement of the skin surface of the suprasternal fossa results in changes in the cross sectional area enclosed by the loop 12, and hence the inductance of the loop, as the portion of the loop on the suprasternal fossa moves relative to the remainder of the loop. As is more fully explained above, changes in the loop inductance are monitored by the oscillator circuitry 14 and demodulating circuit 18, and displayed on the CRT 23 and/or strip chart recorder 25. Consequently, the voltage signal as displayed on the CTR 23 or recorded on the strip chart recorder 25 is indicative of the extent of movement of the skin surface of the suprasternal fossa. By comparing this signal with intrapleural pressure as measured by an esophageal balloon catheter, it was found that at zero flow i.e. at end inspiration and end expiration, there is a linear relationship between intrapleural pressure and movement of the surface of the suprasternal fossa. This is illustrated in Fig. 5 wherein intrapleural pressure as measured with an esophageal balloon catheter is plotted vs. the signal amplitude recorded on the strip chart recorder 25 for a single subject at various tidal volumes with a respiration rate in the range of approximately 15—20 breaths per minute. The graph illustrates a linear relationship as exemplified by the line 32 approximating the plotted points. The plotted points in Fig. 5 were recorded at zero flow, which was determined by simultaneous recordings on a spirometer. Alternatively, zero flow points may be non-invasively determined from the respiration volume signal recorded on an apparatus of the type disclosed in British Patent No. 1 596 298.

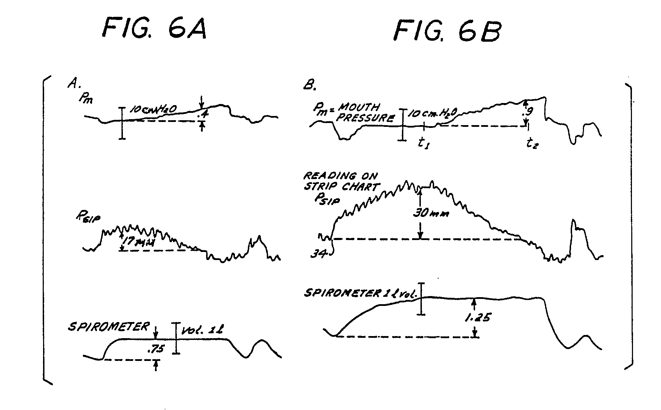

By employing alternative techniques for monitoring intrapleural pressure, the output signal from the apparatus 10 may be calibrated during an initial calibration procedure, whereby subsequent readings will indicate actual intrapleural pressure at zero flow rates. For example, the output signal from the demodulating circuit 18 may be calibrated by comparison with intrapleural pressure measurements as simultaneously recorded with an esophageal balloon catheter. In another calibration technique, the subject is fitted with a mouthpiece which accommodates unencumbered inspiration, but interrupts flow completely upon expiration. The output signal from the apparatus 10 is first observed at the end expiratory level, which may be determined from simultaneous measurements taken with a spirometer. This level is indicated at the point 34 on the graph of Fig. 6B. The subject is then instructed to first inspire, and then expire. During expiration, the mouth pressure is recorded from a pressure tap in the mouthpiece. The mouth pressure required during expiration to return the output signal from the system 10 to the previous end expiratory level is then equivalent to intrapleural pressure. This is designated the "null technique". Thus, and referring to Fig. 6B, calibration is effected by equating the output signal change over the interval from peak inspiration at time $t_1$ back to end expiratory level at time $t_2$ with the mouth pressure change over the same interval. The same technique is illustrated in Fig. 6A for a smaller tidal volume. It should be noted that the resulting calibration is only effective with relatively immobile subjects, and that movement of the head or neck affects the accuracy of the calibrated signal.

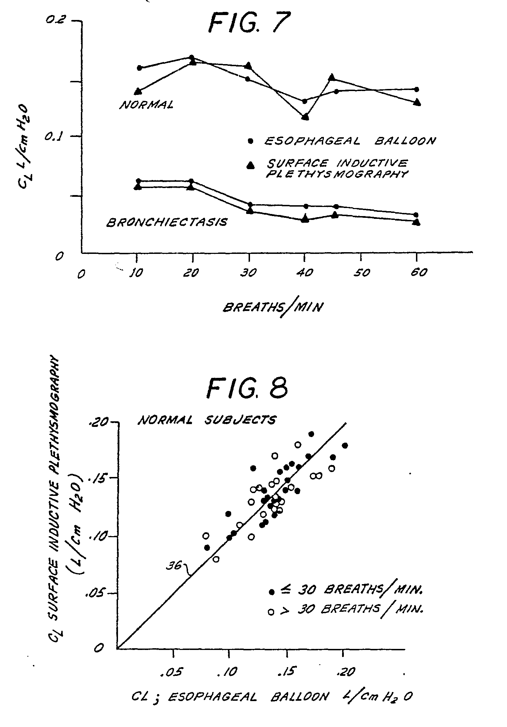

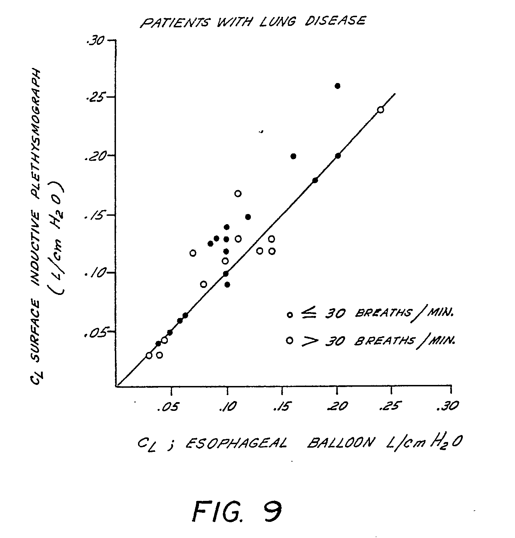

Once the apparatus 10 is calibrated for a particular subject 24, that subject's lung compliance $C_L$ may be determined. Lung compliance is defined by the relationship $\Delta V/\Delta P$ where $\Delta V$ is the change in tidal volume over a given time interval, and $\Delta P$ is the change in intrapleural pressure over the same interval. With the system 10 calibrated, $\Delta P$ at zero flow rates is readily determined. $\Delta V$ may be determined by employing a spirometer, or the non-invasive system disclosed in British Patent No. 1 596 298. Lung compliance is clinically useful as it has been observed that lung compliance is reduced in patients with pulmonary congestion, edema, fibrosis, and is elevated during asthmatic attacks and in emphysema. By recording lung compliance at various breathing frequencies the dependence of lung compliance on breath frequency, i.e. dynamic lung compliance, may be calculated. This is illustrated in Fig. 7, wherein lung compliance at zero flow for both normal and lung diseased patients is recorded at respiration rates of 15, 20, 30, 40, 45 and 60 breaths per minute. Also plotted on Fig. 7 is dymanic lung compliance as simultaneously recorded with an esophageal balloon catheter. The close correlation is apparent. The accuracy of dynamic lung compliance readings recorded with the surface inductive plethysmograph of the invention is further apparent from the line approximations 36 in Figs. 8 and 9 wherein lung compliance for a plurality of subjects at varying breath rates based on intrapleural pressure measurements from the apparatus 10 is plotted vs. lung compliance based on simultaneous intrapleural pressure measurements taken with an esophageal balloon catheter. The data for Fig. 8 was recorded from normal patients, whereas the Fig. 9 data was recorded from patients with lung disease. Dynamic lung compliance is clinically significant as it has been observed that lung compliance decreases with increasing breath frequency when the smaller airways of the lungs are obstructed such as occurs during early lung disease in smokers.

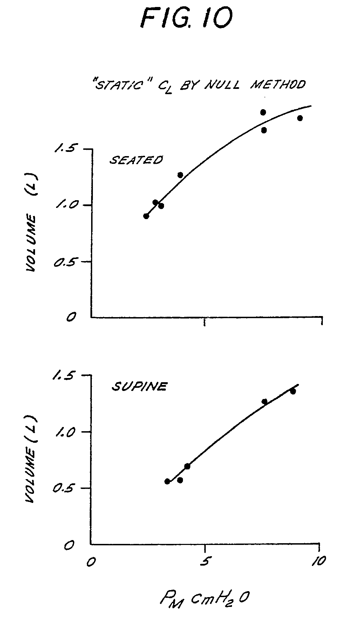

The system 10 may also be utilized to calculate "static" lung compliance. This is accomplished by recording lung compliance for a subject at different tidal volumes for a substantially zero respiration rate. Because intrapleural pressure and tidal volume are not truly linearly related with increasing tidal volumes, it is desirable to determine intrapleural pressure for each different tidal volume by mouth pressure measurements in accordance with the technique more fully described above. Tidal volume, L, vs. mouth pressure, $P_M$ (=intrapleural pressure) for increasing tidal volumes for a subject in the seated and supine positions is plotted in Fig. 10. As may be seen from Fig. 10, this technique yields consistent results for both positions. This is especially significant as intrapleural pressure measurements recorded with an esophageal balloon catheter tend to be inaccurate in the supine position.

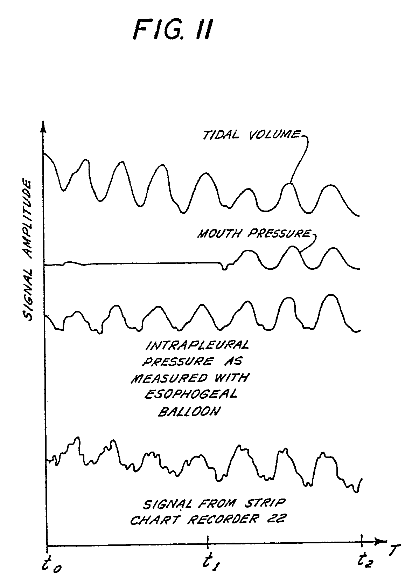

It was also found that intrapleural pressure as measured in accordance with the present invention is accurately reflected even during elastic and non elastic loading of the respiratory system. This is illustrated in the case of elastic loading by the graph of Fig. 11. In Fig. 11, tidal volume as recorded by the apparatus disclosed in said U.S. Patent No. 4,308,872, mouth pressure, intrapleural pressure as recorded with an esophageal balloon catheter, and intrapleural pressure as recorded by the apparatus 10 of the present invention are all plotted vs. time for a particular subject. The subject is initially breathing normally, and it may be seen that during this interval ($t_0$—$t_1$) intrapleural pressure as recorded with the apparatus 10 parallels intrapleural pressure changes as recorded with an esophageal balloon catheter. At time $t_1$, the subject's respiratory tract is elastically loaded by having the subject breathe against a closed volume. This is indicated by both a decrease in tidal volume, and a gradual increase in mouth pressure over the interval $t_1$—$t_2$. During the interval $t_1$—$t_2$, it may be seen that intrapleural pressure as measured with the apparatus 10 of the present invention still parallels intrapleural pressure as measured with the esophageal balloon catheter.

Even if the apparatus 10 of the present invention is not calibrated, the qualitative signal at the output of the demodulating circuit 18 is still useful. For example, obstructive apnea is characterized by increased negative intrapleural pressures, which may be qualitatively determined when monitoring is effected on a long term basis. In this regard, it will be apparent to those skilled in the art that the microprocessor illustrated in Fig. 3 may be programmed to detect amplitude and/or frequency variations and to provide an alarm signal upon detection of particular conditions such as apnea.

The apparatus of the present invention has applications apart from measuring intrapleural pressure. For example, the apparatus of the invention may be used to monitor the activity of the accessory muscles of respiration, which are located on either side of the suprasternal space. To do so, one portion of the loop 12 is disposed on the skin surface over the sternomastoid muscle. As the muscle contracts and relaxes the loop is spread apart and narrowed, respectively, such that mechanical activity of the muscle is detected. As these muscles are only minimally active during normal breathing at rest, but exhibit increased contraction with rapid breathing, airway obstruction, and increased respiratory center output, the detection of their activity is clinically useful. Further, this technique is free of electrical artifacts that can occur when activity is monitored indirectly as with the moving time window average of surface electromyography.

Still other possible applications of the apparatus of the present invention include, but are not limited to, measuring: movement of hinge joints, flaring of the nasal alae during labored breathing; blinking; engorgement of the penis or clitoris during sleep; opening and closing of the mouth; wrinkling of the skin of the face; motion of the intercostal spaces of the rib cage during breathing; motion of the abdominal muscles during breathing; monitoring of skin turgor; and monitoring of changes in the amount of ascitic fluid in the abdomen. Proper placement of the loop 12 for each of the above applications will be apparent to those skilled in the art once this description is known. Possible invasive applications include monitoring movement of particular surface areas of the heart and lungs.

Since the scope of the present invention is intended to incorporate various changes and modifications to the preferred apparatus described hereinabove, this description should be construed as illustrative, and not in a limiting sense, the scope of the invention being defined by the following claims.

**Claims**

1. Apparatus (10) for measuring movement of a portion of the skin surface of a subject (24) said apparatus comprising:

a conductive loop (12) dimensioned for disposition on the skin surface without enveloping the subject,

means (14, 18) connected to said loop for generating a signal indicative of the inductance of said loop,

characterised in that the loop is adapted such that at least a portion thereof may be secured to the skin surface of the suprasternal fossa of the subject whereby movement of said skin surface causes corresponding movement of said portion of the loop in such a way that the cross-sectional area enclosed by the loop is changed.

2. Apparatus as claimed in Claim 1 characterised in that the signal generating means comprises a variable frequency LC oscillator (14) wherein said loop comprises the inductive element, said oscillator converting changes in said inductance to corresponding changes in the frequency of the oscillator output signal.

3. Apparatus as claimed in Claim 2 characterised in that it includes means (18) for converting changes in the frequency of said oscillator output signal to corresponding changes in signal amplitude.

4. Apparatus as claimed in any preceding claim characterised in that the remainder of said loop is adapted for disposition on the manubrium of the sternum.

5. Apparatus as claimed in any preceding claim characterised in that said loop has a circumference of about twelve centimeters.

6. Apparatus as claimed in any preceding claim characterised in that said loop is approximately elliptically shaped, the major axis of said ellipse being approximately six centimeters, and the minor axis being approximately one and a half centimeters.

7. Apparatus as claimed in any preceding Claim characterised in that it further comprises means for calibrating said signal to provide actual intrapleural pressure.

8. Apparatus as claimed in Claim 7 characterised in that it further comprises means for comparing said signal with intrapleural pressure as measured with an esophageal balloon catheter at zero flow, and means for adjusting said signal to equal said intrapleural pressure as measured with said esophageal balloon catheter.

9. Apparatus as claimed in any preceding claim, characterised in that it further comprises means for recording the value of said signal at end expiration, means for incorporating said subject's respiratory tract in a closed system prior to expiration, means for monitoring during expiration the airway pressure of said subject required to return said signal to said end expiration value, said required airway pressure equalling actual intrapleural pressure, and optionally means for adjusting said signal to equal said required airway pressure.

10. Apparatus as claimed in Claim 9 characterised in that said means for monitoring airway pressure comprises means for monitoring mouth pressure.

11. Apparatus as claimed in Claim 7 characterised in that it further comprises means for monitoring said subject's tidal volume at zero flow, means for simultaneously monitoring said subject's actual intrapleural pressure, and means for dividing tidal volume by the corresponding intrapleural pressure at zero flow at a plurality of respiration rates whereby to obtain dynamic lung compliance.

12. Apparatus as claimed in any one of Claims 1 to 6 further comprising means for monitoring said subject's tidal volume at zero flow for increasing tidal volumes, means for recording the value of said signal at end expiration, means for incorporating said subject's respiratory tract in a closed system prior to expiration, and means for monitoring during expiration the airway pressure of said subject required to return said signal to said end expiration value, said required airway pressure equalling actual intrapleural pressure and means for dividing tidal volume at zero flow by the corresponding actual intrapleural pressure for increasing tidal volumes whereby to obtain static lung compliance.

13. A modification of the apparatus as claimed in any one of Claims 1 to 3, characterised in that said loop is adapted and dimensioned such that at least a portion thereof may be secured to the skin surface over the sternomastoid muscle, said signal thereby being indicative of the activity of the accessory muscles of respiration.

## Patentansprüche

1. Vorrichtung (10) zum Messen einer Bewegung eines Abschnitts der Hautoberfläche eines lebenden Objekts (24), welche aufweist:

eine leitfähige Schleife (12), die derart dimensioniert ist, daß sie auf der Hautoberfläche angeordnet wird, ohne das Objekt zu umhüllen,

eine mit der Schleife verbundene Einrichtung (14, 18) zum Erzeugen eines Signals, welches die Induktanz der Schleife anzeigt,

dadurch gekennzeichnet, daß die Schleife so ausgebildet ist, daß mindestens ein Abschnitt davon an der Hautoberfläche der Fossa suprasternalis des Objekts befestigt werden kann, wodurch eine Bewegung der Hautoberfläche eine entsprechende Bewegung des Abschnitts der Schleife derart verursacht, daß die von der Schleife umfaßte Querschnittsfläche geändert wird.

2. Vorrichtung nach Anspruch 1, dadurch gekennzeichnet, daß die Signalerzeugungseinrichtung einen LC-Oszillator (14) mit variabler Frequenz aufweist, wobei die Schleife das induktive Element aufweist, und wobei der Oszillator Änderungen der Induktanz in entsprechende Änderungen der Frequenz des Ausgangssignals des Oszillators umwandelt.

3. Vorrichtung nach Anspruch 2, gekennzeichnet durch eine Einrichtung (18) zum Umwandeln von Änderungen der Frequenz des Ausgangssignals des Oszillators in entsprechende Änderungen der Signalamplitude.

4. Vorrichtung nach einem der vorhergehenden Ansprüche, dadurch gekennzeichnet, daß der Rest der Schleife derart ausgebildet ist, daß er auf dem Manubrium des Sternums angeordnet werden kann.

5. Vorrichtung nach einem der vorhergehenden Ansprüche, dadurch gekennzeichnet, daß die Schleife einen Umfang von etwa 12 cm aufweist.

6. Vorrichtung nach einem der vorhergehenden Ansprüche, dadurch gekennzeichnet, daß die Schleife etwa elliptisch geformt ist, wobei die große Achse der Ellipse etwa 6 cm und die kleine Achse der Ellipse etwa 1,5 cm lang ist.

7. Vorrichtung nach einem der vorhergehenden Ansprüche, ferner gekennzeichnet durch eine Einrichtung zum Kalibrieren des Signals zum Liefern des tatsächlichen intrapleuralen Drucks.

8. Vorrichtung nach Anspruch 7, ferner gekennzeichnet durch eine Einrichtung zum Vergleichen des Signals mit dem intrapleuralen Druck, wie er mit einem ösophagealen Ballonkatheter bei Nullströmung gemessen wird, und eine Einrichtung zum Anpassen des Signals, so daß es dem intrapleuralen Druck gleich ist, wie er mit dem ösophagealen Ballonkatheter gemessen wird.

9. Vorrichtung nach einem der vorhergehenden Ansprüche, ferner gekennzeichnet durch eine Einrichtung zum Aufzeichnen des Werts des Signals am Ende des Ausatmens, eine Einrichtung zum Inkorporieren der Atemwege der Objekts in ein geschlossenes System vor dem Ausatmen, eine Einrichtung zum Überwachen des Drucks der Luftwege des Objekts während das Ausatmens, welcher erforderlich ist, um das Signal auf den Wert am Ende des Ausatmens zurückzubringen, wobei der erforderliche Luftwegsdruck gleich dem tatsächlichen intrapleuralen Druck ist, und gegebenenfalls eine Einrichtung zum Anpassen des Signals, so daß es dem erforderlichen Luftwegsdruck gleich ist.

10. Vorrichtung nach Anspruch 9, dadurch gekennzeichnet, daß die Einrichtung zum Überwachen des Luftwegsdrucks eine Einrichtung zum Überwachen des Munddrucks aufweist.

11. Vorrichtung nach Anspruch 7, ferner gekennzeichnet durch eine Einrichtung zum Überwachen des Atemvolumens des Objekts bei Nullströmung, eine Einrichtung zum gleichzeitigen Überwachen des tatsächlichen intrapleuralen Drucks des Objekts, und eine Einrichtung zum Teilen des Atemvolumens durch den entsprechenden intrapleuralen Druck bei Nullströmung bei mehreren Atmungsgeschwindigkeiten, um dadurch die dynamische Lungendehnbarket zu erhalten.

12. Vorrichtung nach einem der Ansprüche 1 bis 6, welche ferner aufweist: eine Einrichtung zum Überwachen des Atemvolumens des Objekts bei Nullströmung zum Erhöhen der Atemvolumina, eine Einrichtung zum Aufzeichnen des Werts des Signals am Ende des Ausatmens, eine Einrichtung zum Inkorporieren der Atemwege des Objekts in ein geschlossenes System vor dem Ausatmen, und eine Einrichtung zum Überwachen des Drucks der Luftwege des Objekts vor dem Ausatmen, welcher erforderlich ist, damit das Signal auf den Wert am Ende des Ausatmens zurückkehrt, wobei der erforderliche Luftwegsdruck gleich dem tatsächlichen intrapleuralen Druck ist, und eine Einrichtung zum Teilen des Atemvolumens bei Nullströmung durch den entsprechenden tatsächlichen intrapleuralen Druck, um die Atemvolumina zu erhöhen, wodurch die statische Lungendehnbarkeit erhalten wird.

Modifikation der Vorrichtung gemäß einem der Ansprüche 1 bis 3, dadurch gekennzeichnet, daß die Schleife derart ausgelegt und dimensioniert ist, daß mindestens ein Abschnitt davon über dem Sternmastoidmuskel an der Hautoberfläche befestigt werden kann, wobei das Signal dadurch die Aktivität der akzessorischen Atmungsmuskeln anzeigt.

## Revendications

1. Dispositif (10) servant à mesurer le déplacement d'une partie de la surface de la peau d'un sujet (24), ledit dispositif comprenant:

une boucle conductrice (12) dimensionnée de manière à pouvoir être disposée sur la surface de la peau sans envelopper le sujet,

des moyens (14, 18) raccordés à ladite boucle pour produire un signal indicative de l'inductance de cette boucle,

caractérisé en ce que la boucle est adaptée de manière qu'au moins l'une de ses parties peut être fixée à la surface de la peau des fosses sus-sternales du sujet, ce qui a pour effet que le déplacement de ladite surface de la peau entraîne un déplacement correspondant de ladite partie de la boucle d'une manière modifiant la surface en coupe transversale entourée par la boucle.

2. Dispositif selon la revendication 1, caractérisé en ce que les moyens de production de signaux comprennent un oscillateur LC à fréquence variable (14), dont l'élément inducteur est contenu dans ladite boucle, ledit oscillateur transformant des modifications de ladite inductance en des modifications correspondantes de la fréquence du signal de sortie de l'oscillateur.

3. Dispositif selon la revendication 2, caractérisé en ce qu'il inclut des moyens (18) servant à convertir des modifications de la fréquence dudit signal de sortie de l'oscillateur en des modifications correspondantes de l'amplitude du signal.

4. Dispositif selon l'une quelconque des revendications précédentes, caractérisé en ce que le reste de ladite boucle est adapté de manière à pouvoir être placé sur le manubrium du sternum.

5. Dispositif selon l'une quelconque des revendications précédentes, caractérisé en ce que ladite boucle possède une circonférence égale à environ douze centimètres.

6. Dispositif selon l'une quelconque des revendications précédentes, caractérisé en ce que ladite boucle possède une forme approximativement elliptique, le grand axe de ladite ellipse possédant une longueur d'environ six centimètres et le petit axe possédant une longueur égale à environ un centimètre et demi.

7. Dispositif selon l'une quelconque des revendications précédentes, caractérisé en ce qu'il comporte en outre des moyens pour étalonner ledit signal de manière à fournir une pression intrapulmonaire réelle.

8. Dispositif selon la revendication 7, caractérisé en ce qu'il comporte en outre des moyens pour comparer ledit signal à une pression intrapulmonaire mesurée à l'aide d'un cathéter à ballon oesophagien, pour un écoulement nul, et des moyens pour régler ledit signal de manière à le rendre égal à ladite pression intrapulmonaire telle que mesurée à l'aide dudit cathéter à ballon oesophagien.

9. Dispositif selon l'une quelconque des revendications précédentes, caractérisé en ce qu'il comporte en outre des moyens pour enregistrer la valeur dudit signal lors de l'expiration finale, des moyens pour incorporer ladite trachée-artère du sujet dans un système fermé avant l'expiraton, des moyens pour contrôler, pendant l'expiration, la pression des voies aériennes dudit sujet, nécessaire pour ramener ledit signal à ladite valeur d'expiration finale, ladite pression requise, présente dans les voies aériennes, étant égale à la pression intrapulmonaire réelle, et facultativement des moyens pour régler ledit signal de manière à le rendre égal à ladite pression requise, présente dans les voies aériennes.

10. Dispositif selon la revendication 9, caractérisé en ce que lesdits moyens servant à contrôler la pression présente dans les voies aériennes comprennent des moyens pour contrôler la pression à l'intérieur de la bouche.

11. Dispositif selon la revendication 7, caractérisé en ce qu'il comporte en outre des moyens pour contrôler ledit volume respiratoire du sujet pour un écoulement nul, des moyens pour contrôler simultanément ladite pression intrapulmonaire réelle du sujet et des moyens pour diviser le volume respiratoire par la pression intrapulmonaire correspondante pour un écoulement nul, et ce pour une pluralité de fréquences respiratoires, de manière à déterminer la compliance dynamique des poumons.

12. Dispositif selon l'une quelconque des revendications 1 à 6, comprenant en outre des moyens pour contrôler ledit volume respiratoire du sujet pour un écoulement nul, pour des volumes respiratoires croissants, des moyens pour enregistrer la valeur dudit signal lors de l'expiration finale, des moyens pour incorporer ladite trachée-artère du sujet dans une système fermé avant l'expiration, et des moyens pour contrôler, pendant l'expiration, la pression présente dans les voies aériennes dudit sujet, nécessaire pour ramener ledit signal à ladite valeur lors de l'expiration finale, ladite pression requise dans les voies aériennes étant égale à la pression intrapulmonaire réelle, et des moyens pour diviser le volume respiratoire pour un écoulement nul, par la pression intrapulmonaire réelle correspondante pour des volumes respiratoires croissants, ce qui permet de déterminer la compliance statique des poumons.

13. Modification du dispositif selon l'une quelconque des revendications 1 à 3, caractérisée en ce que ladite boucle est adaptée et dimensionnée de manière qu'au moins l'une de ses parties peut être fixée sur la surface de la peau au-dessus du muscle sternomastoïdien, ledit signal étant de ce fait indicatif de l'activité des muscles respiratoires secondaires.

*FIG. I*

*FIG. 2*

1

FIG. 3.

FIG. 4

FIG. 5

# FIG. 6A

# FIG. 6B

## FIG. 7

## FIG. 8

FIG. 9

## FIG. 10

"STATIC" $C_L$ BY NULL METHOD

## FIG. II

SIGNAL AMPLITUDE

TIDAL VOLUME

MOUTH PRESSURE

INTRAPLEURAL
PRESSURE AS
MEASURED WITH
ESOPHOGEAL
BALLOON

SIGNAL FROM STRIP
CHART RECORDER 22

$t_0$       $t_1$       $t_2$   T